# EUROPEAN PATENT SPECIFICATION

(11) **EP 1 343 790 B1**
(45) Date of publication and mention of the grant of the patent: **01.03.2006**
(21) Application number: 01997475.7
(22) Date of filing: 20.11.2001
(51) Int. Cl.: C07D 501/36

(54) **CEPHALOSPORIN PRODUCTION**
HERSTELLUNG VON CEPHALOSPORIN
PRODUCTION DE CEPHALOSPORINE

(30) Priority: 22.11.2000 US 252707 P
(43) Date of publication of application: 17.09.2003
(73) Proprietor: SANDOZ AG, 4002 Basel (CH)
(72) Inventor: BERGER, Andres, A-6330 Ebbs (AT); DECRISTORFORO, Martin, A-6233 Kramsach (AT); LUDESCHER, Johannes, A-6252 Breitenbach (AT); SCHLEICH, Herbert, A-6250 Kundl (AT)
(74) Representative: Gros, Florent
(86) International application number: PCT/EP2001/013443
(87) International publication number: WO 2002/042266

(56) References cited:
- WO-A-87/01117
- US-A- 5 403 929
- DATABASE WPI Derwent Publications Ltd., London, GB; AN 1993-012142 XP002197985 & JP 04 338332 A (TAKEDA CHEM. IND., LTD.) , 25 November 1992 (1992-11-25)
- DATABASE WPI Derwent Publications Ltd., London, GB; AN 1999-585823 XP002197986 & JP 11 255653 A (TAKEDA CHEM. IND., LTD.) , 21 September 1999 (1999-09-21)

## Description

The present invention relates to cephalosporins, more specifically to ceftiofur of formula 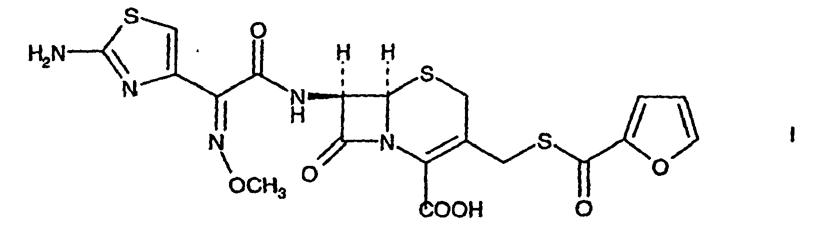 which is {6R-[6α,7β(Z)]}-7-{[(2-amino-4-thiazolyl)(methoxyimino)acetyl]amino}-3-{[(2-furanylcarbonyl)-thio]methyl}-8-oxo-5-thia-1-azabicyclo[4.2.0]oct-2-ene-2-carboxylic acid, e.g. described in The Merck Index, 12th edition, item 1999. Ceftiofur is known as a pharmaceutically active compound, e.g. useful as an antibiotic, such as an antibacterial agent, e.g. used in veterinary medicine, e.g. in the form of a salt with sodium or in the form of a salt with hydrochloric acid. Processes for the production of ceftiofur in free form, in the form of a salt with hydrochloric acid or hydrobromic acid or in the form of a sodium salt are known. A known production process of ceftiofur in the form of a sodium salt (ceftiofur-Na) includes the following steps
a) treating ceftiofur in the form of a salt with hydrochloric or hydrobromic acid in an aqueous organic solvent with polyvinylpyridine, which polyvinylpyridine is activated by treatment with aqueous HCl, rinsing with water, treatment with 10% NaOH solution, rinsing with water and tetrahydrofurane and drying; to obtain a solution comprising ceftiofur in free base form,
b) filtering the solution obtained in step a) to remove the polyvinylpyridine;
c) treating the filtrate obtained in step b) with 2-ethylhexanoic acid in the form of a sodium salt to obtain ceftiofur-Na, and
d) isolating ceftiofur in the form of a sodium salt by precipitation from an aqueous organic solvent, e.g. acetone.

Ceftiofur-Na may be obtained in amorphous form.

We have now surprisingly found a process for the production of pure ceftiofur in the form of a sodium salt, hereinafter designated as "ceftiofur-Na", starting from ceftiofur in the form of a salt with hydrochloric acid or hydrobromic acid, hereinafter designated as "ceftiofur-HCl" or "ceftiofur-HBr", wherein the use of activated polyvinylpyridine may be avoided.

In one aspect the present invention provides a process for the production of ceftiofur in the form of a sodium salt comprising the steps
a) treating ceftiofur in the form of a salt with hydrochloric acid or hydrobromic acid with a sodium source in an aqueous solvent, e.g. water,
b) separating ceftiofur in the form of a sodium salt from sodium chloride or sodium bromide in an aqueous solvent obtained in step a) by use of an appropriate membrane separation system;
c) optionally concentrating the solution obtained in step b), and
d) isolating ceftiofur in the form of a sodium salt from a solution obtained in step b) or, optionally, obtained in step c).

Isolating, e.g. including drying, may be carried out as appropriate, preferably by lyophilisation or spray drying.

A process according to the present invention may be e.g. carried out as follows:
Ceftiofur-HCl or ceftiofur-HBr may be suspended or dissolved in an aqueous solvent, e.g. including water or a mixture of water and an appropriate organic solvent, preferably water. The mixture obtained is treated with an appropriate sodium source, e.g. including a sodium source which is able to convert cefiofur-HCl or ceftiofur-HBr into ceftiofur-Na, such as an inorganic sodium source, e.g. sodium hydroxide, sodium carbonate, sodium bicarbonate. The sodium source may be used in solid form or in solution, e.g. in an aqueous solution. A pH of 5 to 9, preferably of 5 to 7.5, is adjusted in the mixture obtained. Treatment with the sodium source is carried out at appropriate temperatures, e.g. including temperatures of 0°C to about 30°C, preferably of 0°C to 5°C. A solution is obtained comprising ceftiofur-Na beside NaCl or NaBr, which solution optionally may be treated with an adsorbing agent, e.g. including charcoal and/or an adsorber resin, e.g. for decolorizing or clear filtration. The optionally adsorbing-agent-treated solution obtained is subjected to a membrane separation system comprising a membrane which is appropriate for separation of NaCl or NaBr from ceftiofur-Na in an aqueous solution and comprising means for removal of a solution of NaCl or NaBr from said system and for adding fresh water to said system. Appropriate membranes for separation of sodium chloride or sodium bromide from ceftiofur-Na in aqueous solution include nanofiltration membranes having a cutoff of ca. 100 to 500 Dalton, a retention of MgSO₄ of over 90%; e.g. and a retention of sodium chloride of below 80%. Such membranes are known and commercially available, e.g. including a polyamide thinfilm composite on a polysulfone carrier, such as the membranes Nanomax 50® of the firm Millipore; NF 45® or NF 70® of the firm Danish Separation Systems; SR-1®, SR-2® or MPF44® of the firm Koch; or other comparable membranes of the firms Celgard, Nitto Denko or PCI; and including ceramic membranes, e.g. ceramic membranes comprising separating layers from titanium or zirconium oxides and an aluminium oxide carrier material. Said membrane separation system comprises means for removal of a solution of NaCl or NaBr and for adding fresh water to the solution comprising ceftiofur-Na. The membrane separation system is run as appropriate, e.g. a separated solution of sodium NaCl or NaBr is removed from the solution comprising ceftiofur-Na and fresh water is added. Separation is run at appropriate temperatures e.g. including temperatures of 0°C to about 30°C, preferably of 0°C to 5°C. The concentration of the solution of ceftiofur and NaCl or NaBr is not critical but separation is faster in a high concentrated solution compared with a low concentrated solution. Convenient concentrations include concentrations of ceftiofur-Na in water of 1 % to 30%, preferably of 10% to 15% (w/w).
A solution of ceftiofur-Na is obtained which is substantially free of NaCl or NaBr.
Ceftiofur-Na may be isolated from the solution obtained as appropriate, e.g. according to a method as conventional, e.g. including direct Iyophilisation or spray drying of the solution obtained according to the present invention; or, the solution obtained may be concentrated as appropriate e.g. according to a method as conventional, e.g. including solvent evaporation, reversed osmosis prior to isolation of ceftiofur-Na; e.g. the concentrated solution may be subjected to lyophilisation or spray drying.
Prior to isolation of ceftiofur-Na from the solution, but after membrane treatment, a buffer may be added to the (concentrated) solution, e.g. including primary or tertiary sodium or potassium phosphate-buffers.
Ceftiofur-Na in solid, amorphous form may be obtained, substantially free of NaCl or NaBr, e.g. containing NaCl or NaBr, but in an amount of 0.2 % and less, e.g. 0.05% and less.
It is one advantage of the present invention and it is novel, that organic solvent may be avoided in the production of ceftiofur-Na according to the present invention. Organic solvents may be such as ethers, e.g. tetrahydrofurane, carboxylic acid esters, e.g. ethylacetate or ketones, e.g. acetone. Ceftiofur-Na may be thus obtained according to the present invention meeting the reqirements of ICH or Pharmacopoeiias with respect to residual solvents; it is practically free of organic solvent.
If a buffer is added prior to lyophilisation or spray drying to the (concentrated) solution of ceftiofur-Na, a composition is obtained after lyophilisation or spray drying which contains beside ceftiofur-Na additionally buffer substances. These buffer substances and the amount thereof is dependent from the amount and composition of the buffer which is added prior to lyophilisation or spray drying to the (concentrated) solution of ceftiofur-Na.
A process according to the present invention may thus be carried out in water as a solvent and in the absence of organic solvent, e.g. tetrahydrofurane. "Absence of organic solvent" includes, however, residual amounts of organic solvent which may be present and which may originate from a production step in ceftiofur production.

In another aspect the present invention provides a process for the production of ceftiofur in the form of a sodium salt from ceftiofur in the form of a salt with hydrochloride or hydrobromide comprising using water as a solvent in the absence of organic solvent such as e.g. tetrahydrofurane, acetone or ethylacetate.

In another aspect the present invention provides ceftiofur in the form of a sodium salt which is practically free of any organic solvent, such as tetrahydrofurane or acetone.

Ceftiofur in the form of a sodium salt which is practically free of organic solvent includes that it meets not only the Pharmacopoeiiasa respectively ICH limits for residual solvents, but the content of organic solvent(s) is, e.g.considerably, below that of Pharmacopoeiias, or ICH limits, respectively, e.g. containing residual amounts of organic solvent which may be present and which may originate from a production step in ceftiofur production, but no organic solvent from the sodium salt production step.

In another aspect the present invention provides a composition consisting essentially of ceftiofur in the form of a sodium salt, an amount of sodium chloride or sodium bromide, e.g. sodium chloride; which is below 0.2%, but more than zero, and a water residue; and optionally buffer substances.

"Essentially" in that respect means that the compostion contains 95% and more, e.g. 98% and more, or 99% and more, but less than 100% of ceftiofur-Na. If buffer substances are present the composition may contain ceftiofur-Na in an amount of less than 95%, i.e. in such an amount less, which corresponds to the amount of buffer substances present. "A water residue" is meant to be an amount of water which is usually present in a lyophilisate or in a spray dried product, which lyophilisate or in a spray dried product is directly, i.e. without any further means, useful for the production of a solid pharmaceutical composition.

Ceftiofur-Na in solid, amorphous form obtained according to the present invention, e.g. in the form of compositions according to the present invention, is useful in the production of pharmaceutical compositions comprising ceftiofur as a pharmaceutically active compound beside pharmaceutically acceptable excipient(s), e.g. in sterile or non-sterile form.

In another aspect the present invention provides the use of a compound of ceftiofur in the form of a sodium salt, e.g. in solid, amorphous form, as obtained according to the present invention, in the production of a pharmaceutical composition comprising ceftiofur as a pharmaceutically active compound beside pharmaceutically acceptable excipient(s).

Excipient(s) are auxiliaries useful in the production of pharmaceutical compositions.

A pharmaceutical composition comprising ceftiofur as a pharmaceutically active compound beside pharmaceutically acceptable excipient(s), e.g. in sterile or non-sterile form, may be produced as appropriate, e.g. according to a process as conventional, using ceftiofur-Na in solid, amorphous form obtained according to the present invention.

Ceftiofur-Na obtained according to the present invention may be used in the same indications and in the same dosage ranges as ceftiofur-Na provided by a prior art process, e.g. as ceftiofur-Na currently on the market.

In another aspect the present invention provides the use of ceftiofur-Na, e.g. in solid, amorphous form, obtained according to the present invention in sterile form as an antibiotic.

In another aspect the present invention provides a process for the production of a pharmaceutical composition comprising ceftiofur as an active agent, comprising the steps:
a) treating ceftiofur in the form of a salt with hydrochloric acid or hydrobromic acid with a sodium source in an aqueous solvent,
b) separating ceftiofur in the form of a sodium salt from sodium chloride or sodium bromide in an aqueous solvent obtained in step a) by use of an appropriate membrane separation system;
c) optionally concentrating the solution obtained in step b),
d) isolating ceftiofur in the form of a sodium salt obtained in step b) or, optionally obtained in step c), by lyophilisation or spray drying to obtain dry ceftiofur in the form of a sodium salt, and
e) producing a pharmaceutical composition wherein the active agent ceftiofur is represented by dry ceftiofur in the form of a sodium salt obtained in step c), optionally under sterile conditions.

In another aspect the present invention provides a process for the production of ceftiofur of formula I in the form of a sodium salt comprising the steps
a) treating a compound of formula I in the form of a salt with hydrochloric acid or hydrobromic acid with a basic sodium source in aqueous solvent; to obtain a compound of formula I in the form of a sodium salt beside sodium chloride or sodium bromide in an aqueous solvent;
b) separating a compound of formula I in the form of a sodium salt from sodium chloride or sodium bromide in an aqueous solvent obtained in step a) by use of an appropriate membrane separation system; and
c) isolating a compound of formula I in the form of a sodium salt obtained in step b).

In the following example all temperatures are in degree Centigrade and are uncorrected. The following abbreviations are used:
Ceftiofur in the form of a salt with hydrochloric acid = ceftiofur-HCl
Ceftiofur in the form of a salt with sodium = ceftiofur-Na

### Example

500 g of ceftiofur-HCl in 10 I of water are stirred at a temperature of 5 to 10°C. To the mixture obtained an aqueous 5% sodium hydroxide solution is added and a pH of 7.5 is adjusted at a temperature which does not exceed 5°C. The mixture obtained is filtrated, treated with active charcoal and the charcoal is filtrated off. The solution obtained is subjected to a membrane separation system comprising a membrane which is a polyamide thinfilm composite on a polysulfone carrier (NANOMAX 50®, Millipore-membrane) and comprising means for removal of a solution of sodium chloride from the system and for adding fresh water to the system. A back pressure of 30 bar is adjusted. A continuous flow of a solution comprising sodium chloride in isotonic concentration through the membrane is obtained and ceftiofur-Na is substantially complete retained by the membrane. The volume of the solution of ceftiofur-Na is kept approximately constant by addition of fresh water to the system. After a six-fold exchange of the volume of water ceftiofur-Na is practically free of sodium chloride. The solution comprising ceftiofur-Na is concentrated to obtain a concentration of 10% to 30% (w/w) and the concentrate obtained is subjected to lyophilisation. Ceftiofur-Na in amorphous form is obtained. Yield: >90%; content chloride: <0,05%

## Claims

1. A process for the production of ceftiofur in the form of a sodium salt comprising the steps
a) treating ceftiofur in the form of a salt with hydrochloric acid or hydrobromic acid with a sodium source in an aqueous solvent,
b) separating ceftiofur in the form of a sodium salt from sodium chloride or sodium bromide in an aqueous solvent obtained in step a) by use of an appropriate membrane separation system;
c) optionally concentrating the solution obtained in step b), and
d) isolating ceftiofur in the form of a sodium salt from a solution obtained in step b) or, optionally obtained in step c).

2. A process according to claim 1, wherein the aqueous solvent is water.

3. A process according to any one of claims 1 to 2, wherein ceftiofur in the form of a sodium salt is isolated by lyophilisation or spray drying.

4. A composition consisting essentially of ceftiofur in the form of a sodium salt, an amount of sodium chloride or sodium bromide, which is below 0.2%, but more than zero, and a water residue; and optionally buffer substances.

5. A process for the production of a pharmaceutical composition comprising ceftiofur as an active agent, comprising the steps:
a) treating ceftiofur in the form of a salt with hydrochloric acid or hydrobromic acid with a sodium source in an aqueous solvent,
b) separating ceftiofur in the form of a sodium salt from sodium chloride or sodium bromide in an aqueous solvent obtained in step a) by use of an appropriate membrane separation system;
c) optionally concentrating the solution obtained in step b),
d) isolating ceftiofur in the form of a sodium salt obtained in step b) or, optionally obtained in step c), by lyophilisation or spray drying to obtain dry ceftiofur in the form of a sodium salt, and
e) producing a pharmaceutical composition wherein the active agent ceftiofur is represented by dry ceftiofur in the form of a sodium salt obtained in step c), optionally under sterile conditions.

6. A process for the production of ceftiofur in the form of a sodium salt from ceftiofur in the form of a salt with hydrochloride or hydrobromide comprising using water as a solvent in the absence of organic solvents.

7. A process for the production of ceftiofur of formula 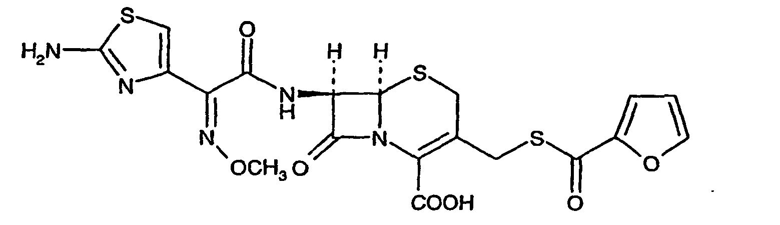
in the form of a sodium salt comprising the steps
a) treating a compound of formula I in the form of a salt with hydrochloric acid or hydrobromic acid with a basic sodium source in an aqueous solvent; to obtain a compound of formula I in the form of a sodium salt beside sodium chloride or sodium bromide in an aqueous solvent;
b) separating a compound of formula I in the form of a sodium salt from sodium chloride or sodium bromide in an aqueous solvent obtained in step a) by use of an appropriate membrane separation system; and
c) isolating a compound of formula I in the form of a sodium salt obtained in step b).

## Patentansprüche

1. Verfahren zur Herstellung von Ceftiofur in Form eines Natriumsalzes, wobei das Verfahren die folgenden Stufen umfasst:
a) Behandlung von Ceftiofur in Form eines Salzes mit Chlorwasserstoffsäure oder Bromwasserstoffsäure mit einer Natriumquelle in einem wässrigen Lösemittel,
b) Trennung von Ceftiofur in Form eines Natriumsalzes von Natriumchlorid oder Natriumbromid in einem wässrigen Lösemittel, das in der Stufe a) erhalten wird, unter Verwendung eines geeigneten Membrantrennsystems,
c) gegebenenfalls Konzentration der in der Stufe b) erhaltenen Lösung und
d) Isolierung von Ceftiofur in Form eines Natriumsalzes aus einer Lösung, die in der Stufe b) erhalten wird, oder gegebenenfalls in der Stufe c) erhalten wird.

2. Verfahren nach Anspruch 1, wobei das wässrige Lösemittel für Wasser steht.

3. Verfahren nach einem der Ansprüche 1 bis 2, wobei Ceftiofur in Form eines Natriumsalzes durch Lyophilisation oder Sprühtrocknung isoliert wird.

4. Zusammensetzung, bestehend im Wesentlichen aus Ceftiofur in Form eines Natriumsalzes, einer Menge an Natriumchlorid oder Natriumbromid, die unterhalb von 0,2 % liegt, aber größer als Null ist, und einen Wasserrückstand; und gegebenenfalls Puffersubstanzen.

5. Verfahren zur Herstellung einer pharmazeutischen Zusammensetzung, die Ceftiofur als wirksames Mittel umfasst, wobei das Verfahren die folgenden Stufen umfasst:
a) Behandlung von Ceftiofur in Form eines Salzes mit Chlorwasserstoffsäure oder Bromwasserstoffsäure mit einer Natriumquelle in einem wässrigen Lösemittel,
b) Trennung von Ceftiofur in Form eines Natriumsalzes von Natriumchlorid oder Natriumbromid in einem wässrigen Lösemittel, das in der Stufe a) erhalten wird, unter Verwendung eines geeigneten Membrantrennsystems,
c) gegebenenfalls Konzentration der in der Stufe b) erhaltenen Lösung, und
d) Isolierung von Ceftiofur in Form eines Natriumsalzes, das in der Stufe b) erhalten wird, oder gegebenenfalls in der Stufe c) erhalten wird, durch Lyophilisation oder Sprühtrocknung, um trockenes Ceftiofur in Form eines Natriumsalzes zu erhalten, und
e) Herstellung einer pharmazeutischen Zusammensetzung, wobei das wirksame Mittel Ceftiofur dargestellt wird durch trockenes Ceftiofur in Form eines Natriumsalzes, das in der Stufe c) erhalten wird, gegebenenfalls unter sterilen Bedingungen.

6. Verfahren zur Herstellung von Ceftiofur in Form eines Natriumsalzes aus Ceftiofur in Form eines Salzes mit Hydrochlorid oder Hydrobromid, wobei das Verfahren umfasst eine Verwendung von Wasser als Lösemittel in Abwesenheit von organischen Lösemitteln.

7. Verfahren zur Herstellung von Ceftiofur der folgenden Formel 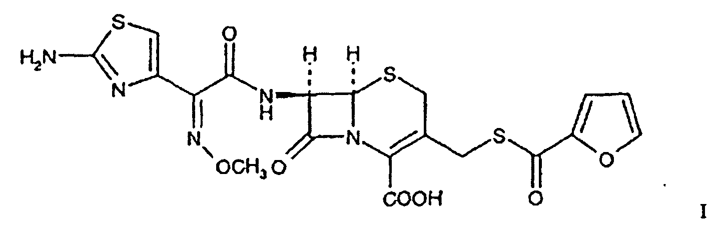
in Form eines Natriumsalzes, wobei das Verfahren die folgenden Stufen umfasst:
a) Behandlung einer Verbindung der Formel I in Form eines Salzes mit Chlorwasserstoffsäure oder Bromwasserstoffsäure mit einer basischen Natriumquelle in einem wässrigen Lösemittel; um eine Verbindung der Formel I in Form eines Natriumsalzes neben Natriumchlorid oder Natriumbromid in einem wässrigen Lösemittel zu erhalten;
b) Trennung einer Verbindung der Formel I in Form eines Natriumsalzes von Natriumchlorid oder Natriumbromid in einem wässrigen Lösemittel, das in der Stufe a) erhalten wird, unter Verwendung eines geeigneten Membrantrennsystems; und
c) Isolierung einer Verbindung der Formel I in Form eines Natriumsalzes, das in der Stufe b) erhalten wird.

## Revendications

1. Procédé pour la production de ceftiofur sous forme d'un sel de sodium, comprenant les étapes de
a) traitement du ceftiofur sous forme d'un sel avec l'acide chlorhydrique ou l'acide bromhydrique, avec une source de sodium dans un solvant aqueux,
b) séparation du ceftiofur sous forme d'un sel de sodium, du chlorure de sodium ou du bromure de sodium dans un solvant aqueux obtenu dans l'étape a) en utilisant un système de séparation par membrane approprié;
c) éventuellement concentration de la solution obtenue dans l'étape b), et
d) isolement du ceftiofur sous forme d'un sel de sodium à partir d'une solution obtenue dans l'étape b) ou, éventuellement, obtenue dans l'étape c).

2. Procédé selon la revendication 1, dans lequel le solvant aqueux est l'eau.

3. Procédé selon l'une quelconque des revendications 1 à 2, dans lequel du ceftiofur sous forme d'un sel de sodium est isolé par lyophilisation ou séchage par pulvérisation.

4. Composition constituée essentiellement de ceftiofur sous forme d'un sel de sodium, d'une quantité de chlorure de sodium ou de bromure de sodium qui est inférieure à 0,2%, mais supérieure à zéro, et d'un résidu d'eau; et éventuellement de substances tampon.

5. Procédé pour la production d'une composition pharmaceutique comprenant du ceftiofur en tant qu'agent actif, comprenant les étapes de:
a) traitement du ceftiofur sous forme d'un sel avec l'acide chlorhydrique ou l'acide bromhydrique, avec une source de sodium dans un solvant aqueux,
b) séparation du ceftiofur sous forme d'un sel de sodium, du chlorure de sodium ou du bromure de sodium dans un solvant aqueux obtenu dans l'étape a) en utilisant un système de séparation par membrane approprié;
c) éventuellement concentration de la solution obtenue dans l'étape b), et
d) isolement du ceftiofur sous forme d'un sel de sodium obtenu dans l'étape b) ou, éventuellement obtenu dans l'étape c), par lyophilisation ou séchage par pulvérisation, pour obtenir du ceftiofur sec sous forme d'un sel de sodium, et
e) production d'une composition pharmaceutique dans laquelle l'agent actif ceftiofur est représenté par du ceftiofur sec sous forme d'un sel de sodium obtenu dans l'étape c), éventuellement dans des conditions stériles.

6. Procédé pour la production de ceftiofur sous forme d'un sel de sodium à partir de ceftiofur sous forme d'un sel avec du chlorhydrate ou du bromhydrate, comprenant l'utilisation d'eau comme solvant en l'absence de solvants organiques.

7. Procédé pour la production de ceftiofur de formule 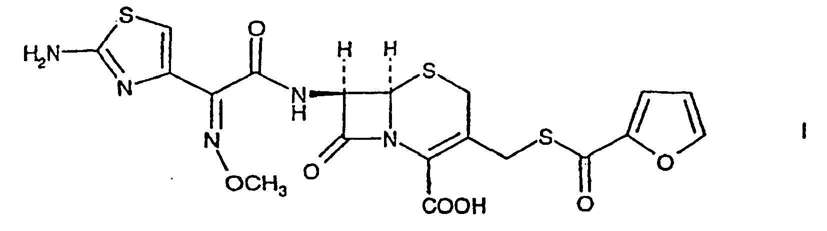
sous forme d'un sel de sodium, comprenant les étapes de
a) traitement d'un composé de formule I sous forme d'un sel avec l'acide chlorhydrique ou l'acide bromhydrique, avec une source de sodium basique dans un solvant aqueux, pour obtenir un composé de formule I sous forme d'un sel de sodium en plus du chlorure de sodium ou du bromure de sodium dans un solvant aqueux;
b) séparation d'un composé de formule I sous forme d'un sel de sodium, du chlorure de sodium ou du bromure de sodium dans un solvant aqueux obtenu dans l'étape a) en utilisant un système de séparation par membrane approprié; et
c) isolement d'un composé de formule 1 sous forme d'un sel de sodium obtenu dans l'étape b).
